# EUROPEAN PATENT APPLICATION

(11) **EP 3 178 368 A1**
(43) Date of publication of application: **14.06.2017**
(21) Application number: 15828986.8
(22) Date of filing: 27.04.2015
(51) Int. Cl.: A61B 1/00

(54) **ANTENNA HOLDER, ANTENNA DEVICE, AND INSPECTION SYSTEM**

(30) Priority: 07.08.2014 JP 2014161786
(71) Applicant: Olympus Corporation, Tokyo 192-8507 (JP)
(72) Inventor: TANAKA, Shinsuke, Hachioji-shi Tokyo 192-8507 (JP)
(74) Representative: Gunzelmann, Rainer
(86) International application number: PCT/JP2015/062740
(87) International publication number: WO 2016/021250

(57) **Abstract**

There are provided an antenna holder and the like allowing antennas to be arranged at appropriate positions on the stomach side and the back side of a subject without much labor. An antenna holder (1A) includes a first antenna accommodating section (10) and a second antenna accommodating section (20) which are separate bodies, and a belt (30) for joining together, and fixing to a subject, the first antenna accommodating section (10) and the second antenna accommodating section (20), where the first antenna accommodating section (10) includes antenna pockets (120a to 120e) arranged on a first base (111) and two belt connection sections (164a, 164b), and where the second antenna accommodating section (20) includes antenna pockets (120f, 120g) arranged on a second base (211) and belt insertion sections (213, 215) through which the belt (30) is to be inserted in a movable manner.

## Description

### Field

The present invention relates to an antenna holder for accommodating an antenna for receiving a signal transmitted from a biological information acquiring apparatus which is inserted into a subject to acquire information about the subject and wirelessly transmits the information, an antenna device, and an examination system. Background

In recent years, a capsule endoscope is known as a biological information acquiring apparatus which is inserted into a subject to acquire information about the subject and wirelessly transmits the information. The capsule endoscope is provided with an imaging function and a wireless communication function inside a casing having a capsule shape, and after it is swallowed by a subject, it captures images while moving inside a digestive tract by peristalsis or the like, and sequentially transmits information of images of the inside of organs of the subject by superimposing the information on wireless signals. The wireless signals are received by an antenna attached to a body surface of the subject, and are taken in by an image processing device such as a workstation and are subjected to predetermined signal processing or image processing, and images of the inside of the subject may thereby be acquired.

As an antenna for receiving wireless signals from the biological information acquiring apparatus, such as the capsule endoscope, a sheet antenna which has an antenna pattern formed on a flexible substrate is used. At the time of attaching the antenna to a subject, the antenna is inserted into a cover provided with an adhesive section, and the cover is directly attached to the body surface of the subject. However, in the case of such an antenna attachment method, although the antenna may be attached to any position on the body surface of the subject, the task requires time, and is burdensome to a user who is to perform the task of attaching the antenna, such as a doctor or a nurse, and to a subject to whom the antenna is to be attached, such as a patient,.

With respect to such a need, Patent Literature 1 discloses a technique of accommodating, in a cloth antenna holder, a sheet antenna having a plurality of antenna circuits formed on one sheet, and wrapping the antenna holder around a subject.

### Citation List

### Patent Literature

Patent Literature 1: JP 5193402 B1

### Summary

### Technical Problem

In the case of observing an organ, such as stomach, which is partially located at a position near the back of a subject, the antenna is desirably arranged also on the back side of the subject so that wireless signals from the biological information acquiring apparatus may be received with high sensitivity. However, in the case of Patent Literature 1, the antenna can be arranged only on the stomach side of the subject.

The present invention has been made in view of the above, and has its object to provide an antenna holder, an antenna device, and an examination system which allow antennas to be arranged at appropriate positions on the stomach side and the back side of a subject without much labor.

### Solution to Problem

To solve the problem described above and to achieve the object, an antenna holder according to the present invention is an antenna holder for accommodating sheet antennas, each sheet antenna on which an antenna circuit receiving a signal from a biological information acquiring apparatus is formed. The biological information acquiring apparatus is configured to: be inserted into a subject to acquire information about the subject; and wirelessly transmit the signal indicating the information. The antenna holder includes: first and second antenna accommodating sections that are separate bodies; and a belt configured to join the first and second antenna accommodating sections together and to fix the first and second antenna accommodating sections to the subject. The first antenna accommodating section includes: a first base; an accommodating pocket arranged on one surface of the first base, provided with an aperture allowing insertion and removal of the antenna, and configured to accommodate the antenna; and two belt connection sections provided at respective end portions of the first antenna accommodating section in a stretching direction of the belt, and connected to respective end portions of the belt. The second antenna accommodating section includes: a second base; an accommodating pocket arranged on one surface of the second base, provided with an aperture allowing insertion and removal of the antenna, and configured to accommodate the antenna; and at least one belt insertion section, arranged on the one surface of the second base, through which the belt is to be inserted in a movable manner.

In the above-described antenna holder, the first antenna accommodating section further includes a cross-shaped first indicator that is provided at a side of the one surface of the first base, and the second antenna accommodating section further includes a rectangular or linear second indicator that is provided at a side of the one surface of the second base.

In the above-described antenna holder, the first antenna accommodating section further includes an openable cover section configured to cover the one surface of the first base, and the first indicator is provided on a surface of the cover section.

In the above-described antenna holder, the two belt connection sections are provided to the cover section.

In the above-described antenna holder, the two belt connection sections are fixed to the cover section by a flat rubber configured to elastically expand and contract in the stretching direction of the belt.

In the above-described antenna holder, the at least one belt insertion section is arranged at a position where, when inserted, the belt covers at least a part of the accommodating pocket provided to the second antenna accommodating section.

In the above-described antenna holder, the antenna includes: a substrate where the antenna circuit is formed; and a connection section configured to connect a cable for transmitting a signal received by the antenna circuit with the antenna circuit, the connection section including a first part configured to sandwich the substrate and a second part whose width is reduced from an end portion of the first part toward the cable, each of the first and second antenna accommodating sections further includes an antenna fixing section configured to fix the antenna accommodated in the accommodating pocket, and the antenna fixing section is formed as a hook and loop fastener, and is provided at a range that covers at least an area where the second part of the connection section is positioned when the antenna is accommodated in the accommodating pocket.

In the above-described antenna holder, the antenna fixing section includes: a first fastener section fixed to the respective first and second antenna accommodating sections; a second fastener section fixed to the respective first and second antenna accommodating sections in a manner being attached to and detached from the first fastener section; and a tab section provided to the second fastener section.

In the above-described antenna holder, the antenna circuit includes a dipole antenna, and a part of the accommodating pocket provided at the first antenna accommodating section and the accommodating pocket provided at the second antenna accommodating section are arranged such that, when the first and second antenna accommodating sections are joined together by the belt, heights of proximal end portions of the antenna circuits formed on the antennas accommodated in the accommodating pockets are aligned with one another.

In the above-described antenna holder, the second antenna accommodating section further includes a belt fixing section configured to fix the second antenna accommodating section to the belt.

In the above-described antenna holder, a first engaging surface forming a part of a hook and loop fastener is provided at the belt, and the belt fixing section is a hook and loop fastener member having a second engaging surface configured to be engaged with the first engaging surface.

In the above-described antenna holder, each of the first and second antenna accommodating sections comprises a plurality of accommodating pockets.

In the above-described antenna holder, the first antenna accommodating section is attached to a stomach side of the subject while a surface opposite the one surface of the first antenna accommodating section is faced to the subject, and the second antenna accommodating section is attached to a back side of the subject while a surface opposite the one surface of the second antenna accommodating section is faced to the subject.

An antenna device according to the present invention includes: the antenna holder; and the antennas accommodated in the accommodating pockets of the first and second antenna accommodating sections.

An examination system according to the present invention includes: the antenna device; the biological information acquiring apparatus configured to: be inserted into the subject to acquire the information about the subject; and wirelessly transmit the signal indicating the information; and a signal processing unit configured to acquire, via the antennas, the signal wirelessly transmitted by the biological information acquiring apparatus and process the signal.

### Advantageous Effects of Invention

According to the present invention, because the first antenna accommodating section and the second antenna accommodating section, where each includes the accommodating pocket for accommodating the antenna, are joined together by the belt, the antennas may be arranged at appropriate positions on the stomach side and the back side of a subject without much labor.

### Brief Description of Drawings

FIG. 1 is a schematic diagram illustrating an example structure of an examination system according to an embodiment of the present invention.
FIG. 2 is a schematic diagram illustrating a state in which an antenna device illustrated in FIG. 1 is attached to a subject.
FIG. 3 is a schematic diagram illustrating a structure of a capsule endoscope illustrated in FIG. 1.
FIG. 4 is a schematic diagram illustrating an outer appearance of the antenna device according to the embodiment of the present invention.
FIG. 5 is a schematic diagram illustrating a structure of a first antenna accommodating section illustrated in FIG. 4.
FIG. 6 is a schematic diagram illustrating a structure of an antenna illustrated in FIG. 5.
FIG. 7 is a schematic diagram for describing a method of placing the antenna in an antenna pocket illustrated in FIG. 5.
FIG. 8 is a schematic diagram illustrating a structure of a cable fixing section illustrated in FIG. 5.
FIG. 9 is a schematic diagram illustrating a state in which a surface cover is removed from a cover section illustrated in FIG. 4.
FIG. 10 is a schematic diagram illustrating a structure of a second antenna accommodating section illustrated in FIG. 4.
FIG. 11 is a schematic diagram illustrating a structure of a belt illustrated in FIG. 4.
FIG. 12 is a schematic diagram illustrating an outer appearance of an antenna device according to a first example modification of the embodiment of the present invention.
FIG. 13 is a schematic diagram illustrating a state, of the antenna device illustrated in FIG. 12, in which a cover section of a first antenna accommodating section is opened.
FIG. 14 is a schematic diagram illustrating a structure of a first antenna accommodating section provided to an antenna device according to a second example modification of the embodiment of the present invention. Description of Embodiments

Hereinafter, an antenna holder, an antenna device, and an examination system according to embodiments of the present invention will be described with reference to the drawings. Additionally, the present invention is not to be limited by the embodiments. It should be noted that the diagrams are only schematic, and the relationship between the thickness and the width of each member, the proportion of the members, and the like differ from reality. The dimensions and the proportions may be different among the drawings. Also, the same components are denoted by the same reference signs in the drawings.

### (Embodiment)

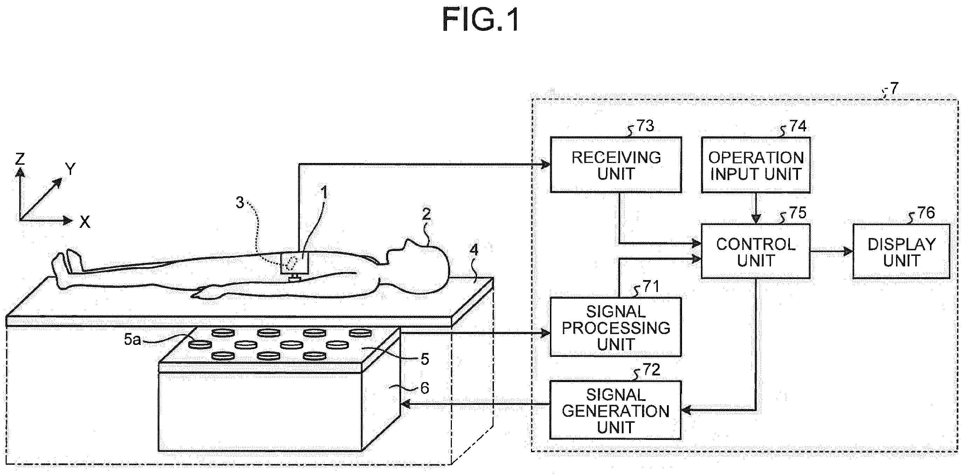

FIG. 1 is a schematic diagram illustrating an example structure of an examination system according to an embodiment of the present invention. As illustrated in FIG. 1, for example, the examination system according to the present embodiment is an examination system for acquiring information mainly about a stomach of a subject, and includes an antenna device 1 to be attached to a subject 2, a capsule endoscope 3 as a biological information acquiring apparatus for being inserted in the subject 2 and for acquiring and wirelessly transmitting information about the inside of the subject 2, a bed 4 where the subject 2 is to lie, a position detection device 5 and a magnetic field generation device 6 provided below the bed 4, and a control device 7 for processing signals received by the antenna device 1 from the capsule endoscope 3, and for performing position detection and position control of the capsule endoscope 3.

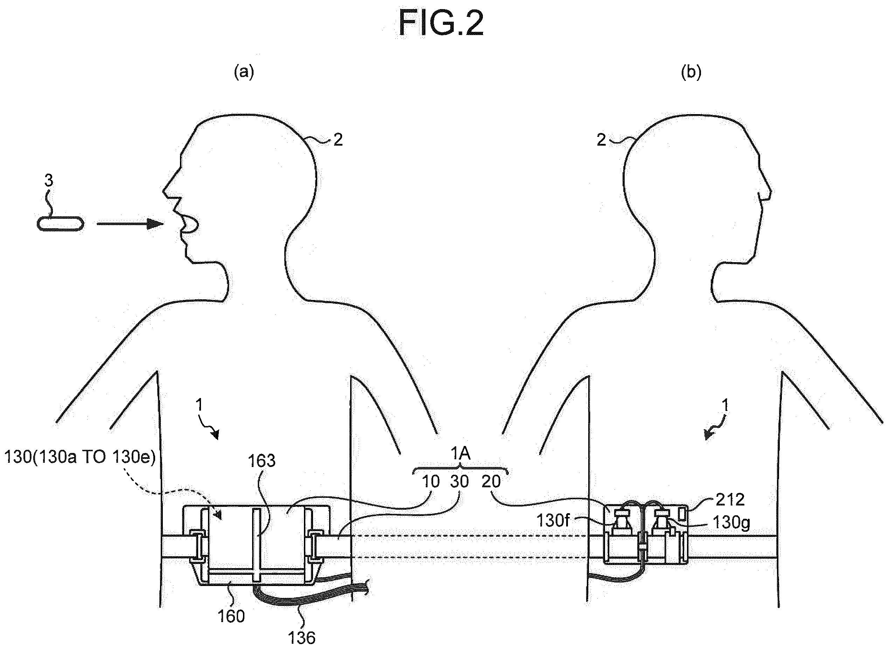

FIG. 2 is a schematic diagram illustrating a state in which the antenna device 1 is attached to the subject 2. The front (stomach side) of the subject 2 is illustrated in (a) of FIG. 2, and the back (back side) of the subject 2 is illustrated in (b) of FIG. 2. As illustrated in FIG. 2, the antenna device 1 includes an antenna holder 1A including a first antenna accommodating section 10 to be attached to the stomach side of the subject 2, a second antenna accommodating section 20 to be attached to the back side of the subject 2, and a belt 30 for joining the first antenna accommodating section 10 and the second antenna accommodating section 20 together and for fixing the first antenna accommodating section 10 and the second antenna accommodating section 20 to the subject 2, and a plurality of antennas 130a to 130g accommodated in the respective first antenna accommodating section 10 and second antenna accommodating section 20. In the following, these antennas 130a to 130g may be collectively referred to as the antenna(s) 130. Such an antenna device 1 receives a signal that is wirelessly transmitted from the capsule endoscope 3, and outputs the received signal to the control device 7. A detailed structure of the antenna device 1 will be described later.

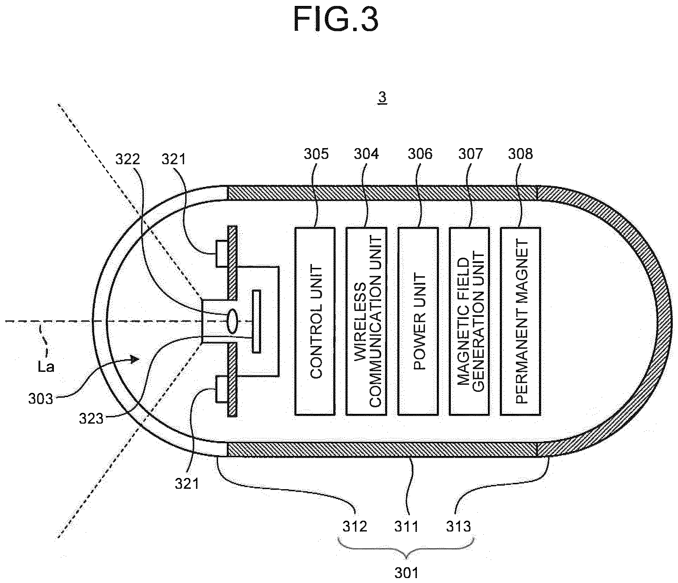

FIG. 3 is a schematic diagram illustrating a structure of the capsule endoscope 3. The capsule endoscope 3 includes, inside a capsule-shaped casing 301, which is an outer package formed to have a size that can be easily inserted into an organ of the subject 2, an imaging unit 303 for capturing the inside of the subject 2 and for generating an image signal, a wireless communication unit 304 for wirelessly transmitting the image signal generated by the imaging unit 303 to the outside, a control unit 305 for controlling each structural unit of the capsule endoscope 3, a power unit 306 for supplying power to each structural unit of the capsule endoscope 3, a magnetic field generation unit 307 for generating an alternating magnetic field for detecting the position of the capsule endoscope 3, and a permanent magnet 308 for enabling magnetic guidance by the magnetic field generation device 6.

The capsule-shaped casing 301 is an outer casing that is formed to have a size that can be inserted into an organ of the subject 2, and includes a cylindrical casing 311 and dome-shaped casings 312, 313, and the apertures on both sides of the cylindrical casing 311 are covered by the dome-shaped casings 312, 313. The dome-shaped casing 312 is a dome-shaped optical member which is transparent to light in a predetermined wavelength band, such as visible light. Also, the cylindrical casing 311 and the dome-shaped casing 313 are colored casings which are substantially opaque to visible light. Such a capsule-shaped casing 301 contains the imaging unit 303, the wireless communication unit 304, the control unit 305, the power unit 306, the magnetic field generation unit 307, and the permanent magnet 308 in a liquid-tight manner.

The imaging unit 303 includes an illumination unit 321, such as an LED, an optical system 322, such as a condenser lens, and an image sensor 323, such as a CMOS image sensor or a CCD. The illumination unit 321 emits illumination light, such as white light, and irradiates an object through the dome-shaped casing 312. The optical system 322 condenses reflected light from the subject 2, and forms an object image on an imaging surface of the image sensor 323. The image sensor 323 generates an image signal indicating an object image in the field of view, that is, an in-vivo image of the subject 2, by photoelectrically converting an optical signal received at the imaging surface.

The wireless communication unit 304 acquires the image signal generated by the imaging unit 303 from the control unit 305, and generates a wireless signal by performing signal processing, such as modulation, on the image signal, and transmits the wireless signal to outside the subject 2.

The control unit 305 controls the operation of the imaging unit 303 and the wireless communication unit 304, and also, controls input and output of signals between these structural units. Specifically, the control unit 305 controls the wireless communication unit 304 to acquire an image signal every time the image sensor 323 generates an image signal and to perform predetermined signal processing on the image signal, and also, to sequentially wirelessly transmit the image signals to outside in chronological order.

The power unit 306 is a battery unit, such as a button-shaped battery or a capacitor, and includes a switch unit, such as a magnetic switch or an optical switch. When in an ON state, the power unit 306 supplies, as appropriate, the power in the battery unit to each structural unit of the capsule endoscope 3, that is, the imaging unit 303, the wireless communication unit 304, the control unit 305, and the magnetic field generation unit 307.

The magnetic field generation unit 307 includes a transmission coil which forms a part of a resonant circuit and which generates a magnetic field when a current flows through, and a capacitor which forms the resonant circuit together with the transmission coil, and generates an alternating magnetic field at a predetermined frequency when supplied with power by the power unit 306.

The permanent magnet 308 is fixedly arranged inside the capsule-shaped casing 301 in such a way that the magnetization direction has a predetermined inclination (for example, 90°) with respect to a long axis La of the capsule-shaped casing 301. Guidance of the capsule endoscope 3 by the magnetic field generation device 6, described later, is realized by the permanent magnet 308 moving according to a magnetic field applied from outside.

Additionally, in the present embodiment, an image signal of an in-vivo image is cited as the information, about the inside of the subject 2, which is to be wirelessly transmitted by the capsule endoscope 3, but other various types of information, such as information about pH inside the subject 2, may also be collected and wirelessly transmitted.

Referring again to FIG. 1, the position detection device 5 includes a plurality of sense coils 5a which are arranged on a planar panel where each sense coil receives an alternating magnetic field generated by the magnetic field generation unit 307 of the capsule endoscope 3 and outputs a detection signal. Each sense coil 5a is formed as a cylindrical coil with a coil spring shape, for example. Such a position detection device 5 is arranged near the subject 2 being examined. FIG. 1 illustrates an example where the position detection device 5 is arranged below the bed 4.

The magnetic field generation device 6 generates a magnetic field for controlling at least one of the position and the posture of the capsule endoscope 3 inserted in the subject 2. Specifically, the magnetic field generation device 6 includes a plurality of electromagnets, and traps the permanent magnet 308 of the capsule endoscope 3 by a combined magnetic field of magnetic fields generated by the electromagnets according to a signal generated by a signal generation unit 72, described later. At this time, the capsule endoscope 3 may be guided to a position or a posture desired by the user by adjusting the magnetic field to be generated by each electromagnet and by changing the combined magnetic field.

The control device 7 includes a signal processing unit 71 for processing a signal output from the position detection device 5, the signal generation unit 72 for generating a signal for operating the magnetic field generation device 6, a receiving unit 73 for receiving a wireless signal transmitted from the capsule endoscope 3 through the antenna device 1, an operation input unit 74 for performing an operation of guiding the capsule endoscope 3, a control unit 75 for controlling the units of the control device 7 in an overall manner and for performing a process of displaying an in-vivo image of the subject 2 based on a wireless signal received by the receiving unit 73, and a display unit 76 for displaying the in-vivo image and other pieces of information.

The signal processing unit 71 takes in a detection signal output from each sense coil 5a of the position detection device 5, adjusts the waveform of the detection signal by a filtering process, and then performs amplification and an A/D conversion process, and then outputs the signal to the control unit 75 as a position detection signal for the capsule endoscope 3.

Under the control of the control unit 75, the signal generation unit 72 generates a drive signal for driving each electromagnet of the magnetic field generation device 6. Additionally, the method of guiding the capsule endoscope 3 is not limited to be performed by the magnetic field generation device 6 and the signal generation unit 72 as described above, and various other known methods may be used. For example, instead of the magnetic field generation device 6, a permanent magnet and driving means for moving and rotating the permanent magnet may be provided outside the subject 2. In this case, the permanent magnet 308 provided inside the capsule endoscope 3 may be trapped by the magnetic field generated by the permanent magnet outside, and the permanent magnet outside may be moved and rotated by the driving means to thereby control the position and the posture of the capsule endoscope 3.

A cable 136 extending from each antenna 130 provided to the antenna device 1 is connected to the receiving unit 73. The receiving unit 73 sequentially takes in, through the cable 136, wireless signals that each antenna 130 received from the capsule endoscope 3. Then, a signal taken in from an antenna with the highest received electric field strength is subjected to predetermined signal processing, such as demodulation processing, and an image signal related to the inside of the subject 2 is acquired and output to the control unit 75.

The operation input unit 74 is an input device used by a user, such as a doctor or a nurse, at the time of performing various input operations, and is configured by a console or the like provided with a keyboard, a mouse, a touch panel, a joystick, various buttons and various switches, for example. The operation input unit 74 outputs a signal according to an operation performed by a user from outside, such as an input operation, to the control unit 75.

The control unit 75 takes in an image signal output from the receiving unit 73, and generates image data for display by performing image processing, such as density conversion such as white balance processing, demosaicking, color conversion processing and gamma conversion, smoothing processing by removal of noise, or sharpening processing by edge enhancement. A position detection signal output from the signal processing unit 71 is taken in, and the position of the capsule endoscope 3 at the time of capturing of an in-vivo image is calculated. Moreover, the control unit 75 outputs a control signal to the signal generation unit 72 according to a signal input from the operation input unit 74, and thereby causes the magnetic field generation device 6 to generate a magnetic field for guiding the capsule endoscope 3. Such a control unit 75 is configured by a workstation, a personal computer or the like.

The display unit 76 is configured by using a liquid crystal display or an organic EL display, for example, and displays an in-vivo image and related information under the control of the control unit 75.

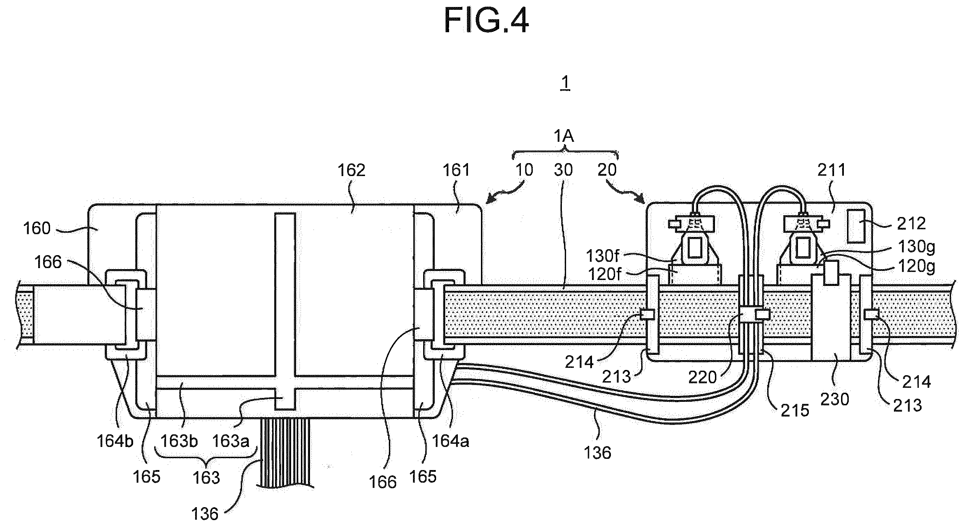

FIG. 4 is a schematic diagram illustrating an outer appearance of the antenna device 1. Additionally, FIG. 4 illustrates a surface which is on the outside (the surface side) at the time of attachment to the subject 2.

As illustrated in FIG. 4, the outer shape of the first antenna accommodating section 10 is a polygonal shape which takes the stretching direction of the belt 30, that is, the direction around the waist of the subject 2, as the longitudinal direction. Specifically, the outer shape of the first antenna accommodating section 10 is a hexagonal shape obtained by diagonally cutting off some of the corners of a substantially rectangular shape or a rectangular shape. Additionally, as will be described later, the outer shape of the first antenna accommodating section 10 is not particularly limited as long a plurality of antennas 130 may be placed inside at predetermined positions. In the following, description will be given taking the longitudinal direction of the first antenna accommodating section 10 as the horizontal direction or a left-right direction, and a direction orthogonal to the longitudinal direction, that is, the height direction of the subject 2, as a vertical direction or a top-down direction.

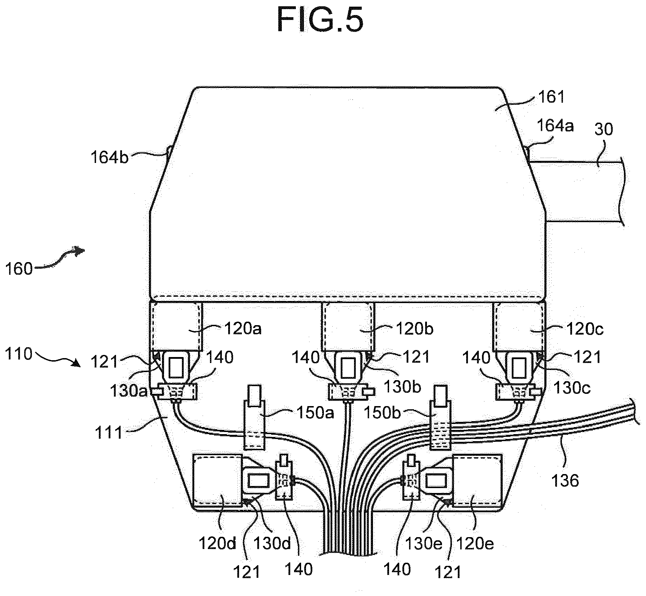

FIG. 5 is a schematic diagram illustrating a structure of the first antenna accommodating section 10. As illustrated in FIG. 5, the first antenna accommodating section 10 includes a main body section 110 to which a plurality (five in FIG. 5) of antennas 130 are to be fixed, and a cover section 160 which is provided to the main body section 110 in a manner capable of being opened and closed. FIG. 5 illustrates a state in which the cover section 160 is open.

As illustrated in FIG. 5, the main body section 110 includes a first base 111, antenna pockets 120a to 120e, provided on the first base 111, for accommodating the respective antennas 130a to 130e, a plurality of antenna fixing sections 140 for fixing edge portions of the respective antennas 130a to 130e accommodated in the respective antenna pockets 120a to 120e, and cable fixing sections 150a, 150b for fixing the cables 136 extending from the respective antennas 130a to 130e at wiring positions on the first base. In the following, the antenna pockets 120a to 120e may be collectively referred to as the antenna pocket(s) 120. Also, the cable fixing sections 150a, 150b may be collectively referred to as the cable fixing section(s) 150.

The first base 111 is made from cloth of nylon or cotton, for example. By using cloth, each antenna 130 may be made to fit the body surface of the subject 2 regardless of the body shape of the subject 2. The type of cloth is not particularly limited, but synthetic fiber, such as nylon, is desirably used. This is because synthetic fiber, such as nylon, is strong and dirt does not easily stick to the same, and also, even if dirt gets stuck, the dirt can be easily removed, thereby allowing the antenna holder 1A to be repeatedly used over a long period of time.

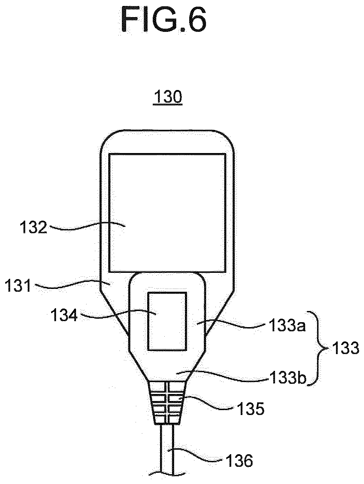

FIG. 6 is a schematic diagram illustrating a structure of the antenna 130. The antenna 130 is a sheet-shaped dipole antenna, and includes a flexible sheet substrate 131, a circuit section 132, provided on the substrate 131, on which an antenna circuit is printed, a connection section 133, fixed to the substrate 131, for connecting a wire drawn out from the circuit section 132 to the cable 136, a label 134 stuck on the surface of the connection section 133, and a protection section 135 for protecting a proximal end portion of the cable 136. The connection section 133 includes a substantially rectangular sandwiching section 133a sandwiching the substrate 131, and a substantially triangular handle section 133b whose width is reduced from an end portion of the sandwiching section 133a toward the protection section 135. That is, the connection section 133 has a shape whose width is more reduced on the side of the cable 136 than on the side of the circuit section 132.

The labels 134 are used as indicators for allowing a user to distinguish among a plurality of antennas 130. Specifically, a user is allow to distinguish among the antennas 130a to 130e by sticking, to the antennas 130a to 130e, the labels 134 of different colors, or the labels 134 on which different numbers or symbols are printed.

The antenna pocket 120 is an accommodating pocket provided with an aperture 121 from which the antenna 130 may be inserted from the distal end of the substrate 131, and like the first base 111, it is made from cloth of nylon or the like. Five antenna pockets 120a to 120e are arranged on the first antenna accommodating section 10. The arrangement of the antenna pockets 120a to 120e, and the directions of the apertures 121 of the antenna pockets 120a to 120e are determined according to the organ, such as the stomach, of the subject 2 who is the examination target of the capsule endoscope 3. Normally, the range in which one antenna 130 can receive wireless signals from the capsule endoscope 3 is smaller than the existing range of the stomach of the subject 2, and thus, the arrangement of the antenna pockets 120 is determined in such a way that the reception ranges of the plurality of antennas 130 are partially overlapped with one another to cover the existing range of the stomach of the subject 2. In the case in FIG. 5, the three antenna pockets 120a to 120c on the upper side are arranged with the apertures 121 facing downward, the antenna pocket 120d on the lower left is arranged with the aperture 121 facing the right side, and the antenna pocket 120e on the lower right is arranged with the aperture 121 facing the left side.

Additionally, display corresponding to the labels 134 stuck to the corresponding antennas 130a to 130e may be shown on the antenna pockets 120a to 120e. Specifically, a label of the same color or with the same number or symbol as the label 134 stuck to each of the antennas 130a to 130e is, for example, sewn and fixed to each of the antenna pockets 120a to 120e for accommodating the corresponding one of the antennas 130a to 130e. A user may thereby surely place each of the antennas 130a to 130e in a predetermined one of the antenna pockets 120a to 120e existing at the position of accommodation.

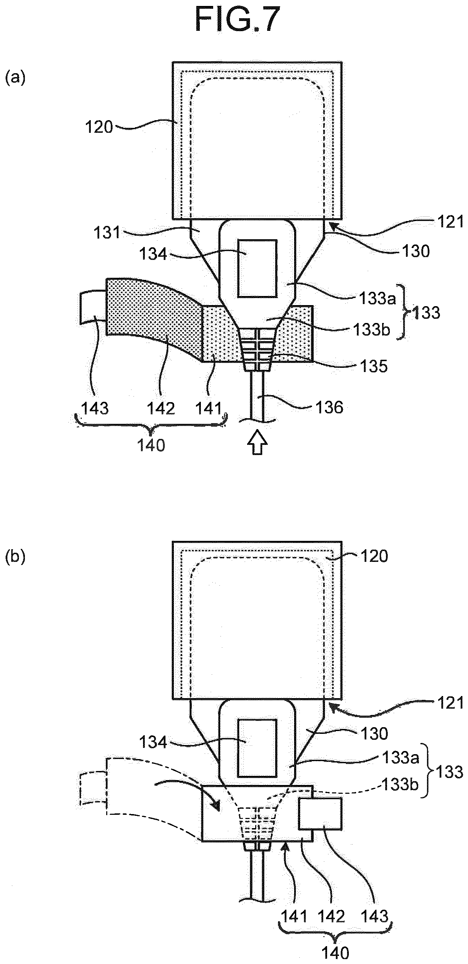

FIG. 7 is a schematic diagram for describing a method of placing the antenna 130 in the antenna pocket 120. The antenna fixing section 140, which is a hook and loop fastener, is provided near the aperture 121 of the antenna pocket 120. The antenna fixing section 140 includes a first fastener section 141 whose surface is almost entirely fixed to the first base 111, a second fastener section 142 whose one end portion is fixed to the first base 111 and which can be freely attached to and detached from the first fastener section 141, and a tab section 143 which is sewn to the other end portion of the second fastener section 142. The tab section 143 is used as a pull at the time of attaching or detaching the second fastener section 142 with respect to the first fastener section 141. Such an antenna fixing section 140 is provided at a range that covers at least the area where the handle section 133b of the connection section 133 is positioned when the antenna 130 is accommodated in the antenna pocket 120. Additionally, in FIG. 7, the antenna fixing section 140 is arranged at a position covering the handle section 133b and the protection section 135.

The first fastener section 141 includes an engaging surface, called a hook surface, which is napped to form hooks, and the second fastener section 142 includes an engaging surface, called a loop surface, which is napped to form loops, for example. The first fastener section 141 and the second fastener section 142 may be coupled together by the engaging surface of the first fastener section 141 and the engaging surface of the second fastener section 142 abutting each other. Additionally, in FIG. 7, the engaging surfaces of the first fastener section 141 and the second fastener section 142 are shaded. Also, the hook and loop fastener is not limited to the type with the hook surface and the loop surface, and various other known types may be used.

As illustrated in (a) of FIG. 7, at the time of placing the antenna 130 in the antenna pocket 120, the second fastener section 142 is peeled off in advance from the first fastener section 141. In this state, the substrate 131 is inserted from the aperture 121 of the antenna pocket 120 until the distal end of the substrate 131 abuts the bottom of the antenna pocket 120. The handle section 133b and the protection section 135 of the connection section 133, that is, the part whose width is reduced toward the cable 136, is arranged on the engaging surface of the first fastener section 141.

Then, as illustrated in (b) of FIG. 7, the second fastener section 142 is closed and is engaged with the first fastener section 141. At this time, the first fastener section 141 and the second fastener section 142 are securely engaged with each other up to the edges of the handle section 133b and the protection section 135 so as to securely fixing the antenna 130 by preventing the connection section 133 from falling out of the antenna fixing section 140 even when the first antenna accommodating section 10 is vertically placed with the aperture 121 of the antenna pocket 120 facing downward.

As illustrated in FIG. 5, the cable fixing section 150a fixes the cable 136 extending from the antenna 130a. Also, the cable fixing section 150b fixes the cable 136 extending from the antenna 130c, and the cables 136 extending from the antennas 130f, 130g provided to the second antenna accommodating section 20 illustrated in FIG. 4. Additionally, the positions and the number of the cable fixing sections 150 are not limited to the example illustrated in FIG. 5. The point is to prevent interference by the cables 136 overlapping the antennas 130 at the first antenna accommodating section 10. Preferably, a relatively long cable 136 extending from the first antenna accommodating section 10 is fixed by the cable fixing section 150, as illustrated in FIG. 5.

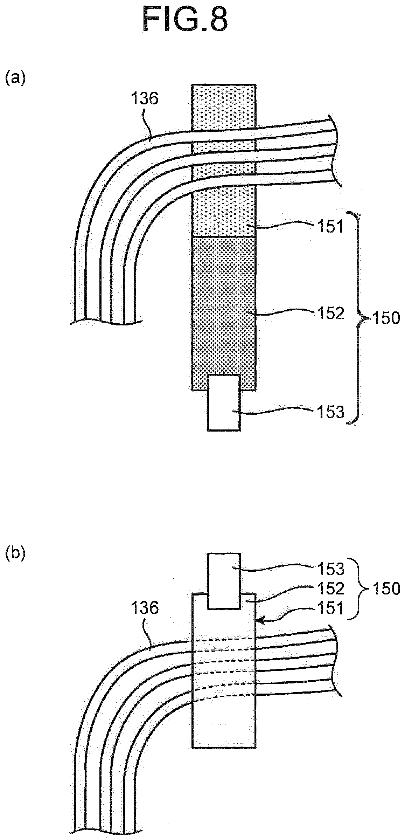

FIG. 8 is a schematic diagram illustrating a structure of the cable fixing section 150. As illustrated in FIG. 8, the cable fixing section 150 includes a first fastener section 151 whose surface is almost entirely fixed to the first base 111, a second fastener section 152 whose one end portion is fixed to the first base 111 and which can be freely attached to and detached from the first fastener section 151, and a tab section 153 which is sewn to the other end portion of the second fastener section 152. The tab section 153 is used as a pull at the time of attaching or detaching the second fastener section 152 with respect to the first fastener section 151. Additionally, in FIG. 8, engaging surfaces of the first fastener section 151 and the second fastener section 152 are shaded.

As illustrated in (a) of FIG. 8, at the time of fixing the cable 136, the cable 136 is arranged on the first fastener section 151 in a state where the second fastener section 152 is peeled off from the first fastener section 151, and then, as illustrated in (b) of FIG. 8, the second fastener section 152 is engaged with the first fastener section 151.

The cover section 160 illustrated in FIGS. 2, 4, 5 and 9 includes a cover base 161 made from cloth of nylon or the like, a surface cover 162 whose upper and lower sides are sewn to the cover base 161, a cross-shaped first indicator section 163 provided to the surface cover 162, and belt connection sections 164a, 164b provided near the right end and the left end of the cover section 160 by support sections 165 and lug sections 166.

The first indicator section 163 illustrated in FIG. 4 is an indicator used for attaching the first antenna accommodating section 10 at an appropriate position on the stomach of the subject 2, and includes a vertical indicator 163a which is provided substantially at the center of the cover section 160 in the horizontal direction and which extends in the vertical direction, and a horizontal indicator 163b which is provided below the center of the cover section 160 in the vertical direction and which extends in the horizontal direction. The color and material of the first indicator section 163 are not particularly limited as long as a patient, i.e. the subject 2, and a user, such as the doctor or the nurse, are allowed to easily identify the first indicator section 163. For example, if the cover section 160 is made from black or gray cloth, the first indicator section 163 may be made from white or fluorescent cloth.

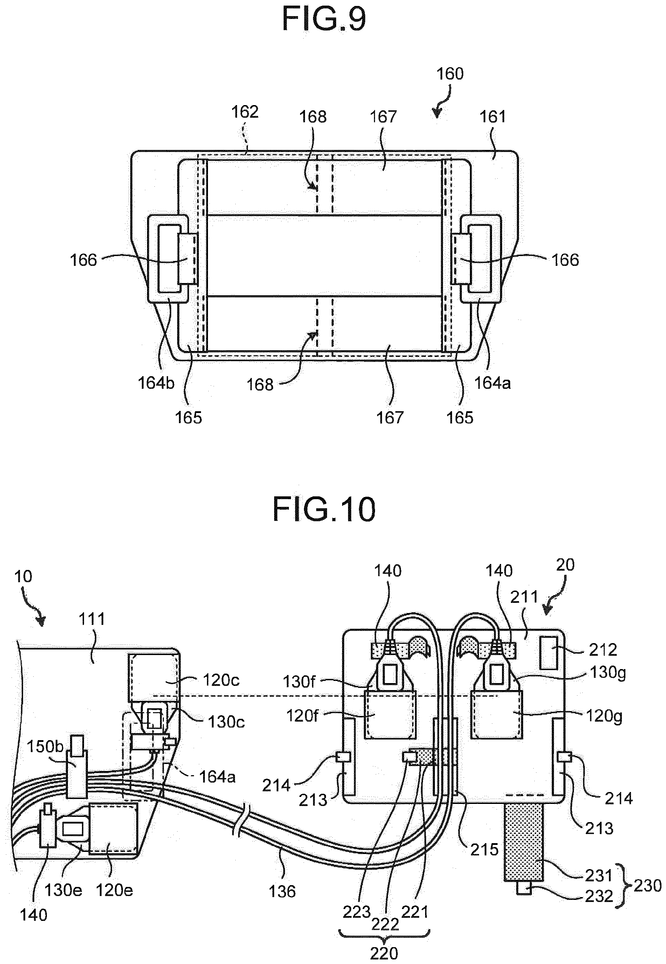

FIG. 9 is a schematic diagram illustrating a state in which the surface cover 162 is removed from the cover section 160. As illustrated in FIG. 9, the two support sections 165 are arranged near the left and right ends of the cover base 161. The lug section 166 around which a part of the belt connection section 164a, 164b is wrapped is sewn to each support section 165. The belt connection sections 164a, 164b are members having a square ring shape and formed of a rigid material such as plastic.

The two support sections 165 are joined together by two flat rubbers 167 that are arranged along the upper side and the lower side of the cover base 161. The flat rubbers 167 are strip-shaped materials which are string-shaped rubbers covered with fiber strings, and may elastically expand and contract in the stretching direction of the string-shaped rubbers (the horizontal direction in FIG. 9). Additionally, the flat rubbers may also be referred to as rubber cords or rubber belts.

Each flat rubber 167 is sewn to the cover base 161 at a center portion 168 in the horizontal direction. That is, the two belt connection sections 164a, 164b are each fixed to the cover base 161 by the lug section 166, the support section 165 and the flat rubbers 167, and may be elastically deformed in the horizontal direction with the center portion 168 as the base point.

Additionally, the number of flat rubbers 167 is not limited to two, and may be one or three or more so long as the left and right support sections 165 may be stably joined and appropriate elasticity may be maintained between the two belt connection sections 164a, 164b.

FIG. 10 is a schematic diagram illustrating a structure of the second antenna accommodating section 20, and illustrates a state in which the belt 30 is removed. As illustrated in FIG. 10, the second antenna accommodating section 20 includes a second base 211 made from cloth of nylon or the like, a plurality (two in FIG. 10) of antenna pockets 120f, 120g provided on the second base 211, antenna fixing sections 140, a second indicator section 212 having a rectangular shape, belt insertion sections 213, 215, a cable fixing section 220 provided on a surface of the belt insertion section 215, and a belt fixing section 230. In the following, the antenna pockets 120f, 120g may be collectively referred to as the antenna pocket(s) 120. Structures of the antenna pockets 120f, 120g, the antennas 130f, 130g to be accommodated in the antenna pockets 120f, 120g, and the antenna fixing sections 140 are the same as those of the antenna pockets 120a to 120e, the antennas 130a to 130e, and the antenna fixing sections 140 provided to the first antenna accommodating section 10. Also, the antenna fixing sections 140, the cable fixing section 220, and the belt fixing section 230 are structured as hook and loop fasteners, and in FIG. 10, engaging surfaces of the hook and loop fasteners are shaded.

The second indicator section 212 is an indicator used for attaching the second antenna accommodating section 20 at an appropriate position on the back of the subject 2, and is attached near a right end portion of the second base 211. The color and material of the second indicator section 212 are not particularly limited as long as a patient, i.e. the subject 2, and a user, such as the doctor or the nurse, are allowed to easily identify the second indicator section 212. For example, if the second base 211 is made from black or gray cloth, the second indicator section 212 may be made from white or fluorescent cloth. Also, the shape of the second indicator section 212 may be a line shape which is long in the vertical direction, instead of being rectangular. In short, it is enough if the position of the second antenna accommodating section 20 in the horizontal direction with respect to the back of the subject 2 may be clearly indicated.

The belt insertion sections 213, 215 form ringshaped apertures with widths allowing insertion of the belt 30 through the second base 211. Of these, the two belt insertion sections 213 are fixed at the lower left and right ends of the second base 211. A tab section 214 is sewn to each belt insertion section 213, and the belt 30 may be easily inserted into the belt insertion section 213 by pulling up the tab section 214 and opening the ring at the time of inserting the belt 30.

The belt insertion section 215 is fixed at substantially a lower center portion of the second base 211 in the horizontal direction, and is fixed by being sewn to the second base 211 at only both end portions. The belt 30 is inserted through the area between the both end portions of the belt insertion section 215 which are fixed to the second base 211.

The cable fixing section 220 is provided on the surface of the belt insertion section 215. The cable fixing section 220 includes a first fastener section 221 whose surface is almost entirely fixed to the surface of the belt insertion section 215, a second fastener section 222 whose one end portion is fixed to the belt insertion section 215 and which can be freely attached to and detached from the first fastener section 221, and a tab section 223 which is sewn to the other end portion of the second fastener section 222. The tab section 223 is used as a pull at the time of attaching or detaching the second fastener section 222 with respect to the first fastener section 221.

As illustrated in FIG. 4, the positions of the belt insertion sections 213, 215 in the vertical direction are determined in such a way that the antenna pockets 120f, 120g are at least partially covered when the belt 30 is inserted through the belt insertion sections 213, 215.

Additionally, the number of belt insertion sections 213, 215 and their positions in the horizontal direction are not particularly limited so long as the second antenna containing section 20 may be stably held when the belt 30 is inserted. For example, one or two wide belt insertion sections may be provided, or four or more belt insertion sections may be provided.

At the time of placing the antennas 130f, 130g in the second antenna accommodating section 20, the antennas 130f, 130g are accommodated in the respective antenna pockets 120f, 120g along a predetermined direction, and the handle sections 133b of the connection sections 133 provided to the antennas 130f, 130g are fixed by the antenna fixing sections 140. Also, the cables 136 extending from the antennas 130f, 130g are fixed together with each other by the cable fixing section 220.

The belt fixing section 230 includes a fastener section 231 having an engaging surface that can be freely attached to and detached from an engaging surface of a hook and loop fastener, described later, provided to the belt 30, and a tab section 232 which is sewn to one end portion of the fastener section 231. The fastener section 231 has the other end sewn to the second base 211, and may be folded over the second base 211.

Next, arrangement of the antenna pockets 120f, 120g at the second antenna accommodating section 20 will be described. In the present embodiment, to mainly observe the stomach of the subject 2 by the capsule endoscope 3, every wireless signal that is transmitted while the capsule endoscope 3 is at the stomach has to be received. Accordingly, the positional relationship, in the vertical direction, of the plurality of antennas 130 arranged on the stomach side and the back side of the subject 2 is specified as follows. That is, as illustrated in FIG. 10, the height of antenna branch points (connection points of the circuit sections 132 and the connection sections 133) of the antennas 130a to 130c arranged on the stomach side and the height of antenna branch points (as described above) of the antennas 130f, 130g arranged on the back side are aligned.

The positions of the antenna pockets 120f, 120g are determined such that, when the belt 30 connected to the belt connection section 164a of the first antenna accommodating section 10 is inserted through the belt insertion sections 213, 215 of the second antenna accommodating section 20, the antenna branch points of the antennas 130a to 130c on the stomach side and the antenna branch points of the antennas 130f, 130g on the back side are horizontally aligned with one another.

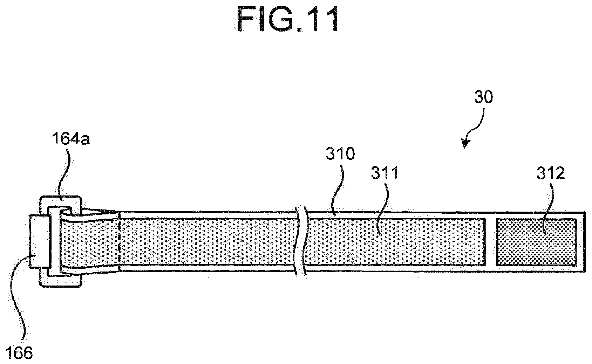

FIG. 11 is a schematic diagram illustrating a structure of the belt 30. As illustrated in FIG. 11, the belt 30 includes, on one surface of a strip-shaped member 310 made from cloth of nylon or the like, a first fastener section 311 which is an engaging surface napped to form hooks, and a second fastener section 312 which is an engaging surface napped to form loops and which can be freely attached to and detached from the first fastener section 311. Of the two, the second fastener section 312 is provided at one end portion of the strip-shaped member 310. Also, the other end portion of the strip-shaped member 310 is connected to the one belt connection section 164a provided to the first antenna accommodating section 10.

Next, a method of attaching the antenna device 1 to the subject 2 will be described. As illustrated in FIGS. 5 and 10, first, the antennas 130 are accommodated in the antenna pockets 120 provided to the first antenna accommodating section 10 and the second antenna accommodating section 20, and the antennas 130 are fixed by the antenna fixing sections 140. Also, the cables 136 extending from the antennas 130 are fixed by the cable fixing sections 150a, 150b, 220.

Next, the cover section 160 of the first antenna accommodating section 10 is closed, and the belt 30 connected to the belt connection section 164a is inserted through the belt insertion sections 213, 215 from an end portion on the second fastener section 312 side.

Next, the intersection point of the cross of the first indicator section 163 is arranged at the position of the navel of the subject 2, and the first antenna accommodating section 10 is placed on the stomach of the subject 2. At this time, the direction of the first antenna accommodating section 10 is adjusted so that the vertical indicator 163a is vertical and the horizontal indicator 163b is horizontal. Then, the belt 30 is wrapped around the waist of the subject 2 while being maintained horizontal.

Then, the second antenna accommodating section 20 is horizontally slid along the belt 30, and the second indicator section 212 is arranged at the position of the spine of the subject 2.

Subsequently, the end portion of the belt 30, on the second fastener section 312 side, is inserted through the ring of the belt connection section 164b and is folded back, and the second fastener section 312 is made to contact the first fastener section 311. At this time, the belt 30 is sufficiently pulled, and the first antenna accommodating section 10 and the second antenna accommodating section 20 are brought into close contact, respectively, with the stomach part and the back of the subject 2.

Furthermore, the second indicator section 212 of the second antenna accommodating section 20 is checked and fine adjustment is performed as necessary, and then, the belt fixing section 230 is folded back and is made to contact the first fastener section 311 of the belt 30. The second antenna accommodating section 20 is thereby fixed to the belt 30.

As described above, according to the present embodiment, a plurality of antennas 130 may be easily and reliably arranged at appropriate positions on the stomach side and the back side of the subject 2. Accordingly, even in a case of observing an organ which is partially on the back side of the subject 2, such as the stomach, signals transmitted by the capsule endoscope 3 may be received by the antenna 130 arranged on the back side of the subject 2 with high sensitivity.

Also, according to the present embodiment, by moving the second antenna accommodating section 20 along the belt 30 and adjusting the gap between the first antenna accommodating section 10 and the second antenna accommodating section 20, the antennas 130 may be arranged at appropriate positions of the subject 2 regardless of the body shape of the subject 2.

Furthermore, according to the present embodiment, because the first antenna accommodating section 10 and the second antenna accommodating section 20 are attached to the subject 2 by the belt 30, the relative position of each antenna 130 with respect to the subject 2 may be maintained even if the subject 2 moves slightly. Particularly, in the present embodiment, because the belt connection sections 164a, 164b are fixed to the cover base 161 by the flat rubbers 167, the first antenna accommodating section 10 and the second antenna accommodating section 20 may be reliably fixed to the subject 2 by tightening the belt 30 in a state where the belt connection sections 164a, 164b are being pulled.

Also, at the first antenna accommodating section 10, the antennas 130 are pressed against the subject 2 by the cover section 160, and thus the reception sensitivity of each antenna 130 for a signal transmitted by the capsule endoscope 3 may be enhanced. At the second antenna accommodating section 20, the belt 30 is arranged on the outside of the antenna pockets 120f, 120g, and thus the antennas 130 are pressed against the subject 2 by the belt 30. Accordingly, also on the back side of the subject 2, the reception sensitivity of each antenna 130 for a signal transmitted by the capsule endoscope 3 may be enhanced.

Moreover, according to the present embodiment, because the antenna fixing section 140 is provided near each antenna pocket 120, the antenna 130 may be prevented from falling out of the antenna pocket 120, and the antenna 130 may be maintained at an appropriate position with respect to the subject 2.

Furthermore, according to the present embodiment, because the cable fixing sections 150 are provided to the first base 111 where the antenna pockets 120 are arranged, interference between the antennas 130 and the cables 136 may be prevented and occurrence of noise may be suppressed.

Furthermore, according to the present embodiment, the second antenna accommodating section 20 is allowed to slide on the belt 30, and to be fixed to the belt 30 by the belt fixing section 230. Accordingly, by sliding and determining the position of the second antenna accommodating section 20 based on the second indicator section 212, and then fixing the second antenna accommodating section 20 to the belt 30 by the belt fixing section 230, the antennas 130 accommodated in the second antenna accommodating section 20 may be maintained at appropriate positions with respect to the subject 2.

### (First Example Modification)

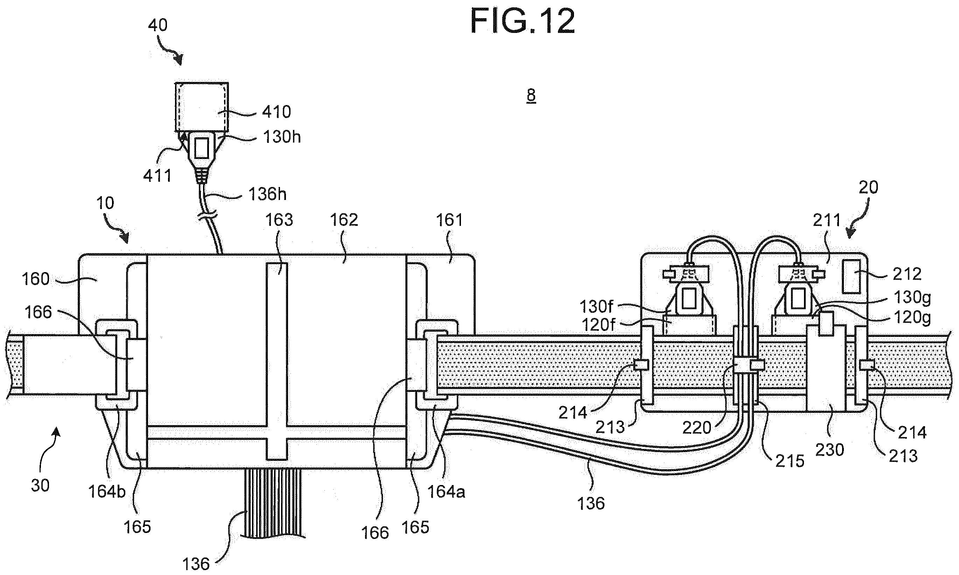

Next, a first example modification of the embodiment of the present invention will be described. FIG. 12 is a schematic diagram illustrating an outer appearance of an antenna device according to the first example modification. As illustrated in FIG. 12, an antenna device 8 according to the first example modification is the antenna device 1 illustrated in FIG. 4 further provided with an antenna section 40 for esophagus for receiving signals transmitted by the capsule endoscope 3 passing through the esophagus. Additionally, the structure of each section of the antenna device 8, other than the antenna section 40 for esophagus, is the same as the structure in the embodiment described above.

The antenna section 40 for esophagus includes an antenna 130h, and an antenna pocket 410 for accommodating the antenna 130h. Of these, the structure of the antenna 130h is the same as the structure of the antennas 130a to 130g accommodated in the first antenna accommodating section 10 and the second antenna accommodating section 20.

As illustrated in FIG. 6, the antenna pocket 410 is an accommodating pocket provided with an aperture 411 from which the antenna 130h may be inserted from the distal end of the substrate 131. An adhesive tape for sticking the antenna pocket 410 to the chest part of the subject 2 is arranged on one surface (in FIG. 12, the surface corresponding to the rear side) of the antenna pocket 410.

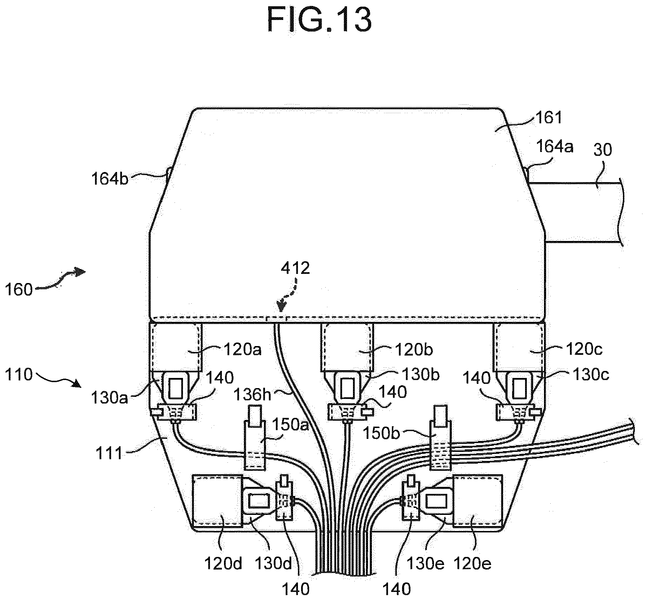

FIG. 13 is a schematic diagram illustrating a state, of the antenna device 8, in which the cover section 160 of the first antenna accommodating section 10 is opened. As illustrated in FIG. 13, a cable 136h extending from the antenna 130h is guided into the first antenna accommodating section 10 through an aperture 412 provided to a part of the cover section 160. The cable 136h is connected to the control device 7 by being bundled with other antennas 130a to 130g.

### (Second Example Modification)

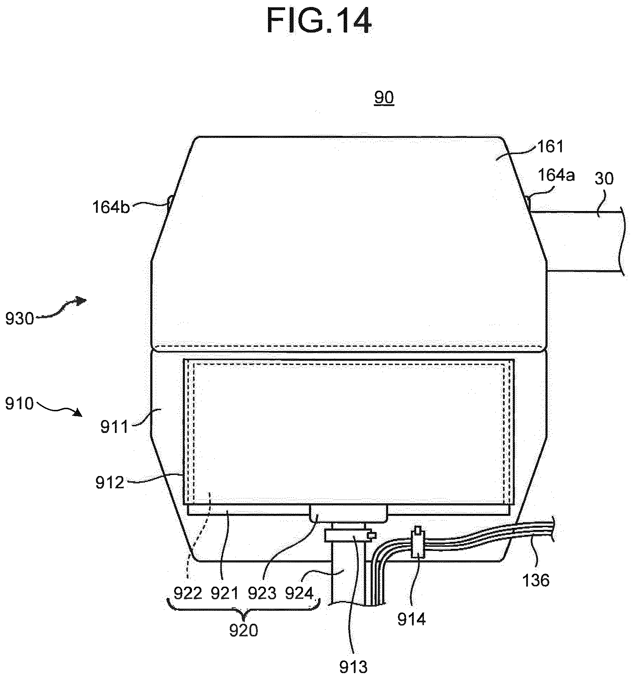

Next, a second example modification of the embodiment of the present invention will be described. FIG. 14 is a schematic diagram illustrating a structure of a first antenna accommodating section provided to an antenna device according to the second example modification. As illustrated in FIG. 14, the antenna device according to the second example modification is provided with a first antenna accommodating section 90 to be attached to the stomach side of the subject 2. Additionally, the structure of the second antenna accommodating section 20 to be attached to the back side of the subject 2 is the same as the one in the above-described embodiment illustrated in FIG. 4.

The first antenna accommodating section 90 includes a main body section 910 and a cover section 930. Of these, the structure of the cover section 930 is the same as the structure of the cover section 160 in the above-described embodiment illustrated in FIGS. 4 and 9. For its part, the main body section 910 includes a first base 911 made from cloth of nylon or the like, one antenna pocket 912 provided on the first base 911, an antenna fixing section 913 provided near the antenna pocket 912, and a cable fixing section 914 for fixing the cables 136 extending from the antennas 130 accommodated in the second antenna accommodating section 20. Of these, the antenna fixing section 913 and the cable fixing section 914 are formed as hook and loop fasteners, like the antenna fixing section 140 and the cable fixing section 150 in the above-described embodiment.

The antenna pocket 912 accommodates one antenna 920 in which a plurality of antenna circuits are formed. The antenna 920 includes a flexible sheet substrate 921, a circuit section 922, provided on the substrate 921, on which a plurality of antenna circuits, each of which is to function as a dipole antenna, are printed, a connection section 923, fixed to the substrate 921, for connecting a wire drawn out from the circuit section 922 to a cable 924, and the cable 924 extending from the connection section 923.

As described, instead of the plurality of antennas 130a to 130e illustrated in FIG. 5, the antenna 920 having a plurality of antenna circuits formed on one substrate 921 is arranged in the first antenna accommodating section 90, and thus, the task of placing the antenna 920 in the first antenna accommodating section 90 may be easily performed in a short time.

In the same manner, an antenna having a plurality of antenna circuits formed on one substrate may be used for the second antenna accommodating section to be attached to the back side of the subject 2, instead of the two antennas 130.

The present invention described above is not limited to the embodiment and the example modifications, and various alterations are possible according to the specifications and the like. For example, the present invention may be configured by removing several structural elements from all the structural elements indicated in the embodiment and the example modifications described above. It is clear from the description given above that various embodiments are possible within the scope of the present invention.

### Reference Signs List

- 1, 8: ANTENNA DEVICE
- 1A: ANTENNA HOLDER
- 2: SUBJECT
- 3: CAPSULE ENDOSCOPE
- 301: CAPSULE-SHAPED CASING
- 303: IMAGING UNIT
- 304: WIRELESS COMMUNICATION UNIT
- 305: CONTROL UNIT
- 306: POWER UNIT
- 307: MAGNETIC FIELD GENERATION UNIT
- 308: PERMANENT MAGNET
- 311: CYLINDRICAL CASING
- 312, 313: DOME-SHAPED CASING
- 321: ILLUMINATION UNIT
- 322: OPTICAL SYSTEM
- 323: IMAGE SENSOR
- 4: BED
- 5: POSITION DETECTION DEVICE
- 6: MAGNETIC FIELD GENERATION DEVICE
- 7: CONTROL DEVICE
- 71: SIGNAL PROCESSING UNIT
- 72: SIGNAL GENERATION UNIT
- 73: RECEIVING UNIT
- 74: OPERATION INPUT UNIT
- 75: CONTROL UNIT
- 76: DISPLAY UNIT
- 10, 90: FIRST ANTENNA ACCOMMODATING SECTION
- 110, 910: MAIN BODY SECTION
- 111, 911: FIRST BASE
- 120, 120a to 120g, 410, 912: ANTENNA POCKET
- 121, 411: APERTURE
- 130, 130a to 130g, 920: ANTENNA
- 131, 921: SUBSTRATE
- 132, 922: CIRCUIT SECTION
- 133, 923: CONNECTION SECTION
- 133a: SANDWICHING SECTION
- 133b: HANDLE SECTION
- 134: LABEL
- 135: PROTECTION SECTION
- 136, 924: CABLE
- 140, 913: ANTENNA FIXING SECTION
- 141, 151, 221, 311: FIRST FASTENER SECTION
- 142, 152, 222, 312: SECOND FASTENER SECTION
- 143, 153, 214, 223, 232: TAB SECTION
- 150, 150a, 150b 220, 914: CABLE FIXING SECTION
- 160, 930: COVER SECTION
- 161: COVER BASE
- 162: SURFACE COVER
- 163: FIRST INDICATOR SECTION
- 163a: VERTICAL INDICATOR
- 163b: HORIZONTAL INDICATOR
- 164a, 164b: BELT CONNECTION SECTION
- 165: SUPPORT SECTION
- 166: LUG SECTION
- 167: FLAT RUBBER
- 168: CENTER PORTION
- 20: SECOND ANTENNA ACCOMMODATING SECTION
- 211: SECOND BASE
- 212: SECOND INDICATOR SECTION
- 213, 215: BELT INSERTION SECTION
- 230: BELT FIXING SECTION
- 231: FASTENER SECTION
- 30: BELT
- 310: STRIP-SHAPED MEMBER
- 40: ANTENNA SECTION FOR ESOPHAGUS
- 412: APERTURE

## Claims

1. An antenna holder for accommodating sheet antennas, each sheet antenna on which an antenna circuit receiving a signal from a biological information acquiring apparatus is formed, the biological information acquiring apparatus being configured to: be inserted into a subject to acquire information about the subject; and wirelessly transmit the signal indicating the information, the antenna holder comprising:
first and second antenna accommodating sections that are separate bodies; and
a belt configured to join the first and second antenna accommodating sections together and to fix the first and second antenna accommodating sections to the subject, wherein
the first antenna accommodating section includes:
a first base;
an accommodating pocket arranged on one surface of the first base, provided with an aperture allowing insertion and removal of the antenna, and configured to accommodate the antenna; and
two belt connection sections provided at respective end portions of the first antenna accommodating section in a stretching direction of the belt, and connected to respective end portions of the belt, and
the second antenna accommodating section includes:
a second base;
an accommodating pocket arranged on one surface of the second base, provided with an aperture allowing insertion and removal of the antenna, and configured to accommodate the antenna; and
at least one belt insertion section, arranged on the one surface of the second base, through which the belt is to be inserted in a movable manner.

2. The antenna holder according to claim 1, wherein
the first antenna accommodating section further includes a cross-shaped first indicator that is provided at a side of the one surface of the first base, and
the second antenna accommodating section further includes a rectangular or linear second indicator that is provided at a side of the one surface of the second base.

3. The antenna holder according to claim 2, wherein
the first antenna accommodating section further includes an openable cover section configured to cover the one surface of the first base, and
the first indicator is provided on a surface of the cover section.

4. The antenna holder according to claim 3, wherein the two belt connection sections are provided to the cover section.

5. The antenna holder according to claim 4, wherein the two belt connection sections are fixed to the cover section by a flat rubber configured to elastically expand and contract in the stretching direction of the belt.

6. The antenna holder according to claim 1, wherein the at least one belt insertion section is arranged at a position where, when inserted, the belt covers at least a part of the accommodating pocket provided to the second antenna accommodating section.

7. The antenna holder according to claim 1, wherein
the antenna includes:
a substrate where the antenna circuit is formed; and
a connection section configured to connect a cable for transmitting a signal received by the antenna circuit with the antenna circuit, the connection section including a first part configured to sandwich the substrate and a second part whose width is reduced from an end portion of the first part toward the cable,
each of the first and second antenna accommodating sections further includes an antenna fixing section configured to fix the antenna accommodated in the accommodating pocket, and
the antenna fixing section is formed as a hook and loop fastener, and is provided at a range that covers at least an area where the second part of the connection section is positioned when the antenna is accommodated in the accommodating pocket.

8. The antenna holder according to claim 7, wherein the antenna fixing section includes:
a first fastener section fixed to the respective first and second antenna accommodating sections;
a second fastener section fixed to the respective first and second antenna accommodating sections in a manner being attached to and detached from the first fastener section; and
a tab section provided to the second fastener section.

9. The antenna holder according to claim 1, wherein
the antenna circuit includes a dipole antenna, and
a part of the accommodating pocket provided at the first antenna accommodating section and the accommodating pocket provided at the second antenna accommodating section are arranged such that, when the first and second antenna accommodating sections are joined together by the belt, heights of proximal end portions of the antenna circuits formed on the antennas accommodated in the accommodating pockets are aligned with one another.

10. The antenna holder according to claim 1, wherein the second antenna accommodating section further includes a belt fixing section configured to fix the second antenna accommodating section to the belt.

11. The antenna holder according to claim 10, wherein
a first engaging surface forming a part of a hook and loop fastener is provided at the belt, and
the belt fixing section is a hook and loop fastener member having a second engaging surface configured to be engaged with the first engaging surface.

12. The antenna holder according to claim 1, wherein each of the first and second antenna accommodating sections comprises a plurality of accommodating pockets.

13. The antenna holder according to claim 1, wherein
the first antenna accommodating section is attached to a stomach side of the subject while a surface opposite the one surface of the first antenna accommodating section is faced to the subject, and
the second antenna accommodating section is attached to a back side of the subject while a surface opposite the one surface of the second antenna accommodating section is faced to the subject.

14. An antenna device comprising:
the antenna holder according to claim 1; and
the antennas accommodated in the accommodating pockets of the first and second antenna accommodating sections.

15. An examination system comprising:
the antenna device according to claim 14;
the biological information acquiring apparatus configured to: be inserted into the subject to acquire the information about the subject; and wirelessly transmit the signal indicating the information; and
a signal processing unit configured to acquire, via the antennas, the signal wirelessly transmitted by the biological information acquiring apparatus and process the signal.
